(19) Europäisches Patentamt
European Patent Office
Office européen des brevets

(11) Publication number: **0 069 527 B1**

# (12) EUROPEAN PATENT SPECIFICATION

(45) Date of publication of patent specification: **21.08.85**

(21) Application number: **82303398.0**

(22) Date of filing: **29.06.82**

(51) Int. Cl.[4]: **C 07 C 149/24,** C 07 C 148/00, A 61 K 31/18, A 61 K 31/16

## (54) Cystine derivatives.

(30) Priority: **07.07.81 GB 8120907**

(43) Date of publication of application: **12.01.83 Bulletin 83/02**

(45) Publication of the grant of the patent: **21.08.85 Bulletin 85/34**

(84) Designated Contracting States: **BE CH LI NL SE**

(56) References cited:

**Chemical Abstracts vol. 67, no. 11, 11 September 1967, Columbus, Ohio, USA D. KOLEV "Hydrogenolysis of some N-substituted amino acids by means of Na in liquid NH3", page 5135, column 1, abstract no. 54422r & Farmatsiya (Sofia), vol. 16, no. 1, 1966, pages 6—10.**

**Chemical Abstracts vol. 66, no. 1, 2 January 1967, Columbus, Ohio, USA E. M. BOYD et al. "The expectorant activity of bisolvon", page 137, column 1, abstract no. 1415n & Arch. Intern. Pharmacodyn. Ther., vol. 163, no. 2, 1966, pages 284—295 (Cat. A, D)**

(73) Proprietor: **RECORDATI S.A. CHEMICAL and PHARMACEUTICAL COMPANY
Corso S. Gottardo 54
CH-6830 Chiasso (CH)**

(72) Inventor: **Nardi, Dante
Via T.Gulli 47
Milan (IT)**
Inventor: **Tajana, Alberto
Via Omboni 3
Milan (IT)**
Inventor: **Motta, Gianni
Via Piave 34/A
Meda Milan (IT)**
Inventor: **Cazzulani, Pietro
Via F. Cavallotti 112 Casal Pusterlengo
Milan (IT)**
Inventor: **Graziani, Gabriele
Via dei Platani 8
Arese Milan (IT)**

(74) Representative: **McKelvey, Ian Edward et al
Serjeants 25 The Crescent
Leicester LE1 6RX (GB)**

**EP 0 069 527 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to cystine derivatives and salts thereof, to methods for their preparation and to pharmaceutical compositions containing them.

The invention provides cystine derivatives having the general formula I

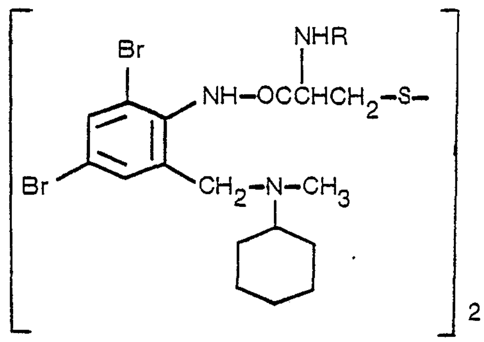

(I)

in which R represents a benzyloxycarbonyl or tosyl group and pharmaceutically acceptable acid addition salts thereof.

The invention further provides a method for the preparation of the cystine derivatives according to the invention, the method comprising reacting a cystyl dichloride of the general formula II

$$Cl—OC—\underset{NHR}{\underset{|}{CH}}—CH_2—S—S—CH_2—\underset{NHR}{\underset{|}{CH}}—CO—Cl \qquad II$$

with 2,4-dibromo-6-(N-methyl-N-cyclohexyl-aminomethyl)-aniline (hereinafter "bromhexine").

The cystyl dichlorides of the general formula II may be obtained from the corresponding acids by any of the conventional methods for conversion of an acid into its chloride, for example by treatment with phosphorus pentachloride in a suitable solvent such as chloroform or diethyl ether. It is not necessary to isolate or purify the dichloride before proceeding with the method according to the invention. A suitable procedure is to precipitate the dichloride from the reaction mixture, for example by addition of ligroin (to diethyl ether solutions) or diethyl ether (to chloroform solutions), filter it off, and add to it a solution of bromhexine in a suitable solvent such as chloroform or ethyl acetate. The reaction with bromhexine proceeds at ambient temperature and may, if desired, be completed under reflux. The resultant cystine derivatives according to the invention may be isolated and purified, and optionally converted into a pharmaceutically acceptable acid addition salt by any of the conventional methods such as direct treatment with the chosen acid in a solvent. Salts of hydrochloric, hydrobromic, sulphuric, nitric, perchloric, fumaric, maleic, phosphoric, glycolic, lactic, salicylic, succinic, toluene-*p*-sulphonic, tartaric, acetic, citric, methan-sulphonic, formic, benzoic, malonic, benzenesulphonic and naphthalene-2-sulphonic acids are preferred.

Cystine derivatives according to the invention and salts thereof possess a good expectorant, antitussive and respiratory tract fluid fluidifying activity, whereas they show a low toxicity. The $LD_{50}$ value, determined in the mouse and in the rat, both i.p., and per os, is greater than 3000 mg/kg for all the tested compounds.

The expectorant activity ($ED_{50}$), determined in the rabbit according to the Boyd method (Boyd and Sheppard, Arch. Int. Pharm. 1966, *163*, 284) was 100 mg/kg (I, R = benzyloxycarbonyl).

The same $ED_{50}$, determined in the mouse according to an improved Mavatari method (Graziani, Cazzulani, Il Farmaco, Ed. Prat., 1981, XXXVI, *3*, 167), was respectively 1.2 mg/kg (I, R = benzyloxycarbonyl) and 3.7 mg/kg (I, R = tosyl).

Compound I (R = benzyloxycarbonyl) showed to be active i.p., as an antitussive agent, at a dose of 30 mg/kg (Method of Charlier, Prost et al., Arch. Int. Pharm., 1961, *134*, 306), through inhalation of citric acid aerosol in the guinea-pig. The activity was determined testing depression of cough.

In order to evaluate the antitussive activity, another procedure was performed. The vagus nerve was stimulated and depression of cough was reported following the injection of the product (Pickering and Jones, Arzn. Forsch., 1979, *29*, 287). The $ED_{50}$ of the compounds I (R = benzyloxycarbonyl and tosyl) was respectively 88 and 74 mg/kg.

Accordingly, the invention also provides a pharmaceutical composition comprising a cystine derivative according to the invention or a salt thereof in admixture with a pharmaceutically acceptable diluent or carrier. The following Examples illustrate the invention.

Example 1

N,N'-ditosyl-cystyl bis[2,4-dibromo-6-(N-methyl-N-cyclohexyl-aminomethyl)-anilide]

To 17.20 g of N,N'-ditosyl-L-cystine dissolved in 185 ml of diethyl ether, 18.5 g of phosphorus pentachloride were slowly added at 20—25°C. The mixture was stirred for 30 minutes and then 650 ml of ligroin were added. The precipitate thus formed was filtered off and to it was added, at 20—25°C, a solution of 27.52 g of bromhexine in 74 ml of chloroform. The mixture was allowed to stand at the above temperature for 36 hours. The whole was extracted with chloroform and washed first with sodium carbonate, then with water. The organic layer was dried with calcium chloride. The solvent was evaporated off and the residue was purified on a silica column, using chloroform:ethyl acetate (3:2 by volume) as eluent. The unreacted bromhexine was separated off. The product, as free base, was dried, dissolved in isopropanol and directly transformed into its hydrochloride by adding hydrogen chloride in isopropanol and then diethyl ether.

The precipitate was crystalled from 95% ethanol and diethyl ether. Mp 191—192°C.

Elemental analysis for $C_{48}H_{60}Br_4N_6O_6S_4 \cdot 2HCl \cdot 2H_2O$

| | C | H | N | Cl | S | Br | $H_2O$ |
|---|---|---|---|---|---|---|---|
| Calculated (%): | 41.96 | 4.66 | 6.11 | 5.16 | 9.33 | 23.26 | 2.76 |
| Found (%): | 41.91 | 4.83 | 5.96 | 5.30 | 9.54 | 23.25 | 2.62 |

Example 2

N,N'-dibenzyloxycarbonyl-cystyl bis[2,4-dibromo-6-(N-methyl-N-cyclohexyl-aminomethyl)-anilide]

To 10.16 g of N,N'-dibenzyloxycarbonyl-L-cystine dissolved in 56 ml of anhydrous chloroform, 10 g of phosphorus pentachloride were slowly added at from −10°C to −5°C.

The reaction mixture was stirred for 10 minutes and then 60 ml of diethyl ether were added. The whole was cooled for one hour. The precipitate thus formed was filtered off and to it was added, at 22°C, a solution of 14.88 g of bromhexine in 40 ml of ethyl acetate. The mixture was stirred at the above temperature for one day and then refluxed for 4 hours.

When the reaction was over, the mixture was extracted with ethyl acetate, washed with sodium carbonate solution, with water, then dried on anhydrous sodium sulphate. The solvent was evaporated off and the residue was treated with hexane. The product was filtered, collected, and crystallized from ethanol. Mp 168°C.

Adding hydrogen chloride in ethanol, the corresponding salt, melting at 195—197°C (with decomposition) was obtained.

Elemental analysis for $C_{50}H_{60}Br_4N_6O_6S_2 \cdot 2HCl$

| | C | H | N | Br | Cl | S |
|---|---|---|---|---|---|---|
| Calculated (%): | 46.28 | 4.81 | 6.47 | 24.63 | 5.46 | 4.94 |
| Found (%): | 45.94 | 4.79 | 6.27 | 24.97 | 5.13 | 4.82 |

Following the same procedures, but varying the acid used for salt formation, the following salts were prepared.

| | |
|---|---|
| Hydrobromide | mp 190—195°C |
| Formate | mp 167—169°C |
| Mandelate | mp 148—149°C |
| Acetate | mp 169—171°C |
| Tartrate | mp 131—134°C |

**Claims**

1. A cystine derivative having the general formula I

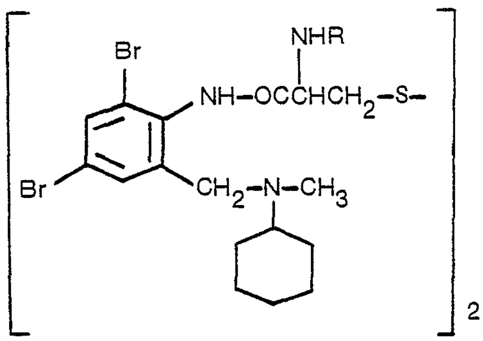

(I)

in which R represents a benzyloxycarbonyl or tosyl group, or a pharmaceutically acceptable acid addition salt of such a cystine derivative.

2. N,N'-ditosyl-cystyl bis[2,4-dibromo-6-(N-methyl-N-cyclohexyl-aminomethyl)-anilide].

3. N,N'-dibenzyloxycarbonyl-cystyl bis[2,4-dibromo-6-(N-methyl-N-cyclohexyl-aminomethyl)-anilide].

4. A method for the preparation of a cystine derivative according to claim 1, the method comprising reacting a cystyl dichloride of the general formula II

$$Cl{-}OC{-}\underset{NHR}{\underset{|}{CH}}{-}CH_2\cdot S{-}S\cdot CH_2{-}\underset{NHR}{\underset{|}{CH}}{-}CO{-}Cl \qquad II$$

with 2,4-dibromo-6-(N-methyl-N-cyclohexyl-aminomethyl)-aniline.

5. A pharmaceutical composition comprising a cystine derivative according to any of claims 1 to 3 or a pharmaceutically acceptable salt thereof in admixture with a pharmaceutically acceptable diluent or carrier.

**Patentansprüche**

1. Ein Cystin-Derivat von der allgemeinen Formel I

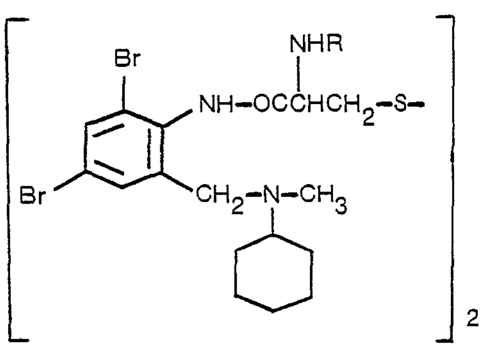

(I)

in welcher R eine Benzyloxycarbonyl- oder Tosyl-Gruppe, oder ein pharmazeutisch annehmbares Säurezusatzsalz eines derartigen Cystin-Derivates darstellt.

2. N,N'-Ditosyl-Cystyl bis[2,4-Dibromo-6-(N-Methyl-N-Cyclohexyl-Aminomethyl)-Anilid].

3. N,N'-Dibenzyloxycarbonyl-Cystyl bis[2,4-Dibromo-6-(N-Methyl-N-Cyclohexyl-Aminomethyl)-Anilid].

4. Verfahren zum Herstellen eines Cystin-Derivates nach Anspruch 1, bei welchem man ein Cystyl-Dichlorid der allgemeinen Formel II

$$Cl{-}OC{-}\underset{NHR}{\underset{|}{CH}}{-}CH_2\cdot S{-}S\cdot CH_2{-}\underset{NHR}{\underset{|}{CH}}{-}CO{-}Cl \qquad II$$

mit 2,4-Dibromo-6-(N-Methyl-N-Cyclohexyl-Aminomethyl)-Anilin wirken lässt.

5. Pharmazeutische Zusammensetzung mit einem Cystin-Derivat nach einem der Ansprüche 1 bis 3 oder ein pharmazeutisch annehmbares Salz von diesem in Mischung mit einem pharmazeutisch annehmbaren Lösungsmittel oder einer Trägersubstanz.

**Revendications**

1. Un dérivé de cystine ayant la formule générale I

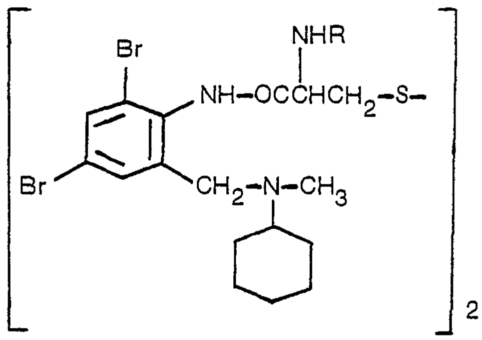

(I)

dans laquelle R représente un groupe de benzyloxycarbonyle ou tosyle ou un sel acide additionnel d'un de ces dérivés de cystine.

2. N,N'-ditosyle-cystyle bis[2,4-dibromo-6-(N-méthyle-N-cyclohéxyle-aminométhyle)-anilide].

3. N,N'-dibenzyloxycarbonyle-cystyle bis[2,4-dibromo-6-(N-méthyle-N-cyclohéxyle-aminométhyle)-anilide].

4. Une méthode pour la préparation d'un dérivé de cystine selon la revendication 1, cette méthode comprenant un dichlorure de cystine de la formule générale II agissant avec

$$Cl{-}OC{-}\underset{NHR}{CH}{-}CH_2\cdot S{-}S\cdot CH_2{-}\underset{NHR}{CH}{-}CO{-}Cl \qquad II$$

2,4-dibromo-6-(N-méthyle-N-cyclohéxyle-aminométhyle)-aniline.

5. Une composition pharmaceutique comprenant un dérivé de cystine selon l'une des revendications 1 à 3 ou un sel de celui-ci, en mélangée à un diluant ou une substance porteuse tous les deux pharmaceutiquement acceptables.